# EUROPÄISCHE PATENTSCHRIFT

(11) **EP 3 582 818 B1**
(45) Veröffentlichungstag und Bekanntmachung des Hinweises auf die Patenterteilung: **04.10.2023**
(21) Anmeldenummer: 18705398.8
(22) Anmeldetag: 15.02.2018
(51) Int. Cl.: A61L 27/36, A61L 27/38

(54) **DEZELLULARISIERTE IMPLANTATHÜLLEN**
DECELLULARISED IMPLANT COVERINGS
ENVELOPPE D'IMPLANT DÉCELLULARISÉE

(30) Priorität: 15.02.2017 DE 102017202429
(43) Veröffentlichungstag der Anmeldung: 25.12.2019
(73) Patentinhaber: Fraunhofer-Gesellschaft zur Förderung der angewandten Forschung e.V., 80686 München (DE); Medizinische Hochschule Hannover, 30625 Hannover (DE)
(72) Erfinder: POHLMANN, Gerhard, 31715 Meerbeck (DE); STIEGHORST, Jan, 29352 Adelheidsdorf (DE); DOLL, Theodor, 30916 Isernhagen (DE)
(74) Vertreter: Altmann Stößel Dick Patentanwälte PartG mbB
(86) Internationale Anmeldenummer: PCT/EP2018/053799
(87) Internationale Veröffentlichungsnummer: WO 2018/149923

(56) Entgegenhaltungen:
- WO-A1-2016/191878
- US-A1- 2011 054 588
- US-A1- 2015 320 911

## Beschreibung

Die Offenbarung betrifft Vorrichtungen und Verfahren zur Verbesserung der Funktionalität von Implantaten. Insbesondere betrifft die Offenbarung ein dezellularisiertes Gewebe biologischen Ursprungs, das zu einer ein Implantat umhüllenden Schicht ausgebildet ist. Weiterhin betrifft die Offenbarung ein Implantat, das von einer Schicht aus dezellularisiertem Gewebe umhüllt ist, sowie die Verwendung eines dezellularisierten Gewebes zur Umhüllung eines Implantats. Schließlich betrifft die Offenbarung ein Verfahren zur Verbesserung der Funktionalität bei gleich bleibender Biokompatibilität eines austauschbaren Implantats umfassend die Schritte des Bereitstellens eines dezellularisierten Gewebes biologischen Ursprungs und das Umhüllen des Implantats mit diesem dezellularisierten Gewebe.

Bei orthopädischen oder stomatologischen Implantaten ist das feste Verwachsen des Implantats mit dem Körper gewünscht und eine Voraussetzung dafür, dass diese Implantate ihre Funktion erfüllen können.

Aktive Funktionsimplantate, z.B. Herzschrittmacher oder Cochleaimplantate, müssen allerdings zu Wartungszwecken gelegentlich explantiert oder gar ganz ausgetauscht werden. In diesen Fällen ist das feste Verwachsen des Implantats mit Gewebe an der Implantationsstelle, z.B. durch fibrinogenes Gewebe (Narbengewebe), unerwünscht und störend.

Bei Biosensoren oder Implantaten mit Elektroden, die Kontakt zum Gewebe herstellen müssen, z.B. Cochleaimplantaten, ist zudem wünschenswert, dass die Elektroden eine hohe Empfindlichkeit über lange Zeit behalten. Der Körper erkennt allerdings gerade solche Implantate als körperfremd und initiiert körpereigene Abwehrreaktionen und die Bildung von Narbengewebe. Beides interferiert mit der Empfindlichkeit und damit der Funktion der Implantate. Für Cochleaimplantate wurden negative Effekte für die Funktion und die Wiederherstellung des Hörvermögens durch Narbengewebe am Implantat ausführlich untersucht. Es wurde vorgeschlagen, die Operationstechniken zu verbessern, um die Bildung von Narbengewebe zu vermindern (Choi 2005). Auch wurde vorgeschlagen, die Narbenbildung am Cochleaimplantat durch Dexamethason zu vermindern, welches vom Implantat freigesetzt werden soll (Wilk 2016).

Bei Cochleaimplantaten kommt andererseits dem Einwachsen von Neuriten zur Elektrode am Implantat eine große Bedeutung zu. Ist die Distanz zwischen Implantat und Gewebe an der Implantationsstelle zu groß, kann es im Rahmen des Einwachsens zu ungewollten und beeinträchtigenden Interaktionen zwischen den Stimulationskanälen kommen (Gstoettner 2001).

Für eine andere Klasse von Implantaten, nämlich die Gewebeimplantate, werden im Stand der Technik dezellularisierte Gewebe verwendet. Dezellularisierte Gewebe wurden bisher vor allem als Gerüststrukturen zur Besiedelung mit Zellen genutzt. Diese besiedelten Gewebe können z.B. in Bereichen wie der Wundheilung, z.B. als Hautimplantate, eine Rolle spielen, aber auch als Herzklappen Verwendung finden (Crapo 2011, Gilbert 2006).

Bei geeigneter Aufbereitung werden dezellularisierte Gewebe kaum oder nicht als Fremdkörper erkannt (Badylak 2011). Dieser Umstand unterstreicht die Eignung für die Herstellung von Herzklappen aus xenogenem oder allogenem dezellularisierten Gewebe (WO2006/122533). Üblicherweise werden die dezellularisieten Gewebe langsam und ohne nennenswerte Kalzifizierung besiedelt (Tudorache 2016).

US2011/054588 A offenbart die Dezellularisierung von Gewebe biologischen Ursprungs. Das dezellularisierte Gewebe wird zusammengerollt, so dass eine umhüllende Schicht in Form eines Schlauches gebildet wird. US2015/320911 A offenbart ein Verfahren für die Herstellung eines dezellularisierten Gewebes, welches durch Behandlung mit physikalischen, chemischen und oder enzymatischen Methoden gewonnen wird. Das dezellularisierte Gewebe wird um einen synthetischen Schlauch (ein implantierbares Ausflussrohr) gewickelt und bildet eine umhüllende Schicht um den synthetischen Schlauch. Das dezellularisierte Gewebe umfasst entzündungshemmend wirksame Substanzen. WO2016/191878 A offenbart ein Cochleaimplantat mit einer umhüllenden Silikontasche.

Es wäre wünschenswert, wenn auch nicht-Gewebeimplantate und insbesondere die austauschbaren, aktiven Implantate so präpariert werden könnten, dass ein Verwachsen mit Gewebe an der Implantationsstelle, z.B. über Narbengewebe, verhindert werden kann. Zudem wäre es vorteilhaft, wenn körpereigene Abstoßungsreaktionen gegen diese Implantate vermieden werden könnten.

Aufgabe der vorliegenden Erfindung ist daher die Bereitstellung von Mitteln und Maßnahmen, die diesen Anforderungen gerecht werden. Die Aufgabe wird durch die in den Patentansprüchen und im Folgenden beschriebenen Ausführungsformen gelöst.

Die Offenbarung betrifft daher ein dezellularisiertes Gewebe biologischen Ursprungs, das zu einer ein Implantat umhüllenden Schicht ausgebildet ist.

Der Begriff "dezellularisiertes Gewebe" betrifft ein Gewebe biologischen Ursprungs, das keine vitalen Zellen mehr aufweist. Solche erfindungsgemäßen, dezellularisierten Gewebe bestehen somit im Wesentlichen aus extrazellulärer Matrix.

Abhängig von der Art des biologischen Ausgangsgewebes kann diese extrazelluläre Matrix eine unterschiedliche Zusammensetzung aufweisen. Typischerweise besteht die extrazelluläre Matrix aus Glykoproteinen und Polysacchariden. Neben diesen beiden Bestandteilen können auch weitere Proteine, z.B. Enzyme, Wachstumsfaktoren, Zytokine, Adhäsionsproteine, Proteoglykane und nicht-Protein Bestandteile wie Glykosaminoglykane in der extrazellulären Matrix vorliegen.

Wichtige Proteine, die in der extrazellulären Matrix vorkommen, sind:
Kollagene, z.B. die fibrilläre Kollagene des Typs I, II, III, V oder XI, die netzbildende Kollagen des Typs IV (in der Basalmembran), VIII oder X, die fibrillenassoziierten Kollagene (FACIT) des Typs IX, XII oder XIV, die perlenschnurartige Kollagene des Typs VI, die Verankerungsfibrillen Kollagen Typ VII und die Kollagene mit Transmembrandomänen des Typs XIII oder XVII,
Elastin,
Fibrillin,
Glykosaminoglykane,
Proteoglykane, oder
Adhäsionsproteine wie Laminine, Fibronektin oder Vitronektin.

Fibrilläre Kollagene, z.B. Kollagen Typ I, II, II, V und XI, verleihen dem Gewebe Zug- und Reisfestigkeit und Steifheit, wohingegen netzbildende Kollagene das Gewebe zusammenhalten und die in die extrazelluläre Matrix eingelagerten Faktoren binden. Neben Kollagenen können auch elastische Fasern in den erfindungsgemäßen, dezellularisierten Geweben vorkommen. Diese Fasern bestehen im Wesentlichen aus den Proteinen Elastin und Fibrillin und vermitteln die Elastizität des Gewebes und die Verformbarkeit. Die extrazelluläre Matrix aus dem Bindegewebe weist z.B. üblicherweise einen sehr hohen Anteil an Kollagenen auf. Dementsprechend ist ein dezellularisiertes Bindegewebe ebenfalls Reich an Kollagenen und weist üblicherweise Eigenschaften auf, die auf die entsprechenden Kollagene zurückgehen.

Bevorzugt ist das dezellularisierte Gewebe zum Umhüllen des Implantats aus dem Gewebe oder Gewebetyp hergestellt worden, der das Implantat in vivo umgeben wird. Soll also ein Implantat in eine Bindegewebeumgebung im Körper eingebracht werden, wird auch das dezellularisierte Gewebe, das als Implantathülle erfindungsgemäß eingesetzt werden soll, ein dezellularisiertes Bindegewebe sein. Es findet also bevorzugt eine isotope oder orthotope Transplantation statt. Bevorzugt kommt das dezellularisierte Gewebe aus der gleichen Spezies, in die auch das Implantat eingebracht werden soll. Hierbei handelt es sich dann bevorzugt um ein allogenes Gewebe. Jedoch können grundsätzlich auch dezellularisierte Gewebe aus anderen Spezies zum Einsatz kommen, sogenannte xenogene Gewebe. Bevorzugt wird bei der Wahl eines geeigneten Ursprungsgewebes für das dezellularisierte Gewebe darauf geachtet, dass das Gewebe mit dem Empfängergewebe, also dem Gewebe, in das das Implantat eingebracht werden soll immunologisch kompatibel ist. Hierzu werden typischerweise die aus der Transplantationsmedizin bekannten Kriterien zur Gewebeverträglichkeit untersucht. Insbesondere sind hyperaktute, akute oder chronische Abstoßungsreaktionen zu verhindern. Besonders bevorzugt kommt das dezellularisierte Gewebe aus dem Individuum, das auch später das umhüllte Implantat erhalten soll, oder es handelt sich um Gewebe von einem nahen Verwandten, z.B. einem einengen Zwilling. Besonders bevorzugt handelt es sich also um autologes oder syngenes Gewebe. Bevorzugt werden menschliche Gewebe verwendet um dezellularisiertes Gewebe im Sinne der Erfindung zu gewinnen, da die meisten Implantate auch bei Menschen zur Anwendung kommen. Trotzdem sind Implantate auch bei Tieren und insbesondere Säugern, wie Haus- und Nutztieren, denkbar.

Verfahren zur Gewinnung von dezellularisieten Geweben aus unterschiedlichen Geweben biologischen Ursprungs sind im Stand der Technik bekannt. Bevorzugt wird das dezellularisierte Gewebe durch Behandlung von Gewebe biologischen Ursprungs mit physikalischen, chemischen und/oder enzymatischen Methoden gewonnen. Ziel der Behandlung ist in jedem Fall das Abtöten der Zellen im Gewebe. Weiterhin können die Zellen auch zerstört und Ihre intrazellulären Bestandteile freigesetzt werden. Neben den physiologisch im Gewebe vorhanden Zellen werden durch die zuvor genannten Behandlungen bevorzugt auch Mikroorgansimen, die im Gewebe als Pathogene vorhanden sein könnten, zerstört. Somit kann vorteilhafterweise auch eine Sterilisation des Gewebes im Rahmen der Dezellularisierung stattfinden. Selbstverständlich kann diese Sterilisation auch separat nach der Dezellularisierung erfolgen, z.B. durch Behandlung mit geeigneten Mitteln zum Inaktivieren von Bakterien, Viren und Pilzen oder durch Temperaturbehandlung.

Die Herstellung des erfindungsgemäß dezellularisierten Gewebes findet bevorzugt unter Reinraumbedingungen entsprechend GMP statt. Die fertigen Implantathüllen werden bevorzugt steril, besonders bevorzugt unter geeigneten Bedingungen in einer Antibiotikalösung, gelagert und bereitgestellt.

Bevorzugte physikalische Methoden zur Gewinnung von dezellularisierten Geweben umfassen Temperatur, Scherkräfte und Druck sowie elektrische Desruption, um Gewebe zu dezellularisieren. So können Zellen z.B. durch schnelles Einfrieren lysiert werden. Hydrostatischer Druck zerstört ebenfalls die Zellen im Gewebe. Üblicherweise wird dieser auch bei erhöhter Temperatur angewendet. Elektroporation ist ein elektrisches Verfahren zur Lyse von Zellen im Gewebe. Bevorzugte Verfahren sind bei Gilbert et al. beschrieben. (Gilbert 2006).

Chemische Methoden zum Abtöten und lysieren der Zellen umfassen bevorzugt die Behandlung des Gewebes mit Detergenzien, Säuren oder Basen. Detergenzien, die bevorzugt verwendet werden, sind ionische, nicht-ionische oder zwitterionische Detergenzien. Nach der Behandlung mit Detergenziene, Säuren oder Basen kann das Gewebe noch mit Enzymen, z.B. Endo- und/oder Exonukleasen, behandelt werden. Detergenz-Behandlungen können zudem mit Säure- oder Base-Behandlungen kombiniert werden. Bevorzugte Verfahren sind bei Crapro et al. beschrieben (Crapo 2011). Besonders bevorzugt kann eine Behandlung mit Detergenzien und osmotisch wirksamen Lösungen eingesetzt werden wie in WO2006/122533 beschrieben.

Bevorzugte enzymatische Methoden zur Gewinnung von dezellularisierten Geweben umfassen die Behandlung von Geweben mit Lipasen, Thermolysin, Galaktosidasen, Nukleasen und/oder Proteasen, insbesondere Trypsin. Typischerweise werden die Zellen des Gewebes hierbei aber zunächst durch z.B. eine Detergenz-Behandlung lysiert. Bevorzugte Verfahren sind ebenfalls bei Crapo et al. beschrieben (Crapo 2011).

Die zuvor genannten Methoden bzw. einzelne Behandlungsschritte können grundsätzlich auch frei miteinander kombiniert werden, dass heißt es gibt Methoden zur Dezellularisierung, die chemische und physikalische oder physikalische und enzymatische oder enzymatische und chemische Methoden oder Verfahrensschritte enthalten. Die Auswahl und Zusammenstellung der Verfahrensschritte zu einer geeigneten Dezellularisierungs-Methode hängt von dem zu dezellularisierenden Gewebe ab und vom Zweck, zu dem das dezellularisierte Gewebe verwendet werden soll. Im Fall der vorliegenden Erfindung ist der Einsatzzweck, die Implantation, d.h. das dezellularisierte Gewebe sollte auf das Gewebe an der Implantationsstelle eingestellt sein.

Das dezellularisierte Gewebe kann bevorzugt mit biologisch aktiven Substanzen versehen werden. Solche biologisch aktiven Substanzen können z.B. das gezielte Einwachsen bestimmter Zellen oder Zellteile ermöglichen, die Bildung von Narbengewebe verhindern oder anti-inflammatorisch wirken. Geeignete Substanzen können somit Wachstumsfaktoren oder immunmodulatorische Zytokine sein. Denkbar sind aber auch Adhäsionsmoleküle oder pharmazeutische Wirkstoffe allgemein.

Besonders bevorzugt können Wachstumsfaktoren für z.B. Nervenzellen verwendet werden, um das Einwachsen von Neuriten in das Gewebe und damit in die Nähe des Implantats zu begünstigen. Immunsuppressive pharmazeutische Wirkstoffe wie Zyklosporin, Tacrolimus, Azathioprin bzw. Mykophenolat, Dexamethason, Glukokortikoide, Basiliximab oder Antithymozytenglobulin-Antikörper können verwendet werden um Abstoßungs- oder Entzündungsreaktionen nach Einbringen des Implantats zu verhindern. Schmerzmittel können ins dezellularisierten Gewebe eingebracht werden, um Schmerzen durch oder nach Implantation zu lindern. Antivirale, antibakterielle oder antimyotische pharmazeutische Wirkstoffe können eigesetzt werden, um eine Infektion nach Implantierung des Implantats zu verhindern. Angiogene Substanzen, z.B. Wachstumsfaktoren, die die Angiogenese begünstigen, können verwendet werden, um Blutgefäße in die Nähe des Implantats zu locken.

Die dezellularisieten Gewebe sollen zu einer ein Implantat umhüllenden Schicht ausgebildet sein. Hierunter ist zu verstehen, dass das dezellularisierte Gewebe zu einer Hüllstruktur ausgeformt sein soll, die das Implantat ganz oder teilweise umhüllen kann. Da es sich jedoch in der Regel um austauschbare Implantate handelt, soll das dezellularisierte Gewebe hierbei nicht komplett auf dem Implantat immobilisiert sein, so dass ein Austausch des Implantats auch dann möglich ist, wenn das dezellularisiete Gewebe mit dem umgebenden Gewebe an der Implantationsstelle nach Einbringen des Implantats verwächst. Besonders bevorzugt umgibt das dezellularisierte Gewebe daher das Implantat als Tasche oder Schlauch. Implantat und dezellularisiertes Gewebe sind dann in der Regel gegeneinander verschiebbar und nicht aneinander fixiert. Das Gewebe kann hierbei als eine kontinuierliche Gewebsschicht das Implantat ganz oder teilweise umhüllen oder regelmäßig oder unregelmäßig angelegte Bereiche aufweisen, in denen kein Gewebe vorhanden ist. Die Hüllstruktur aus dezellularisierten Gewebe kann somit auch ein Netz oder ein netzartiger Schlauch sein oder aus Gewebefragmenten bestehen (Gewebepatches).

Bevorzugt wird zur Anfertigung einer Hüllstruktur zunächst geeignetes Gewebe entnommen, das dann anschließend durch physikalische, chemische und/oder enzymatische Verfahren dezellularisiert wird. Das dezellularisierte Gewebe wird dann mit einem Laser in geeignete Formen zugeschnitten und ggf. mit biologisch aktiven Substanzen wie andernorts hierin beschrieben versehen. Verschiedene Gewebeteile können auch mittels eines Lasers oder Gewebeklebern miteinander verbunden werden, sodass Hüllstrukturen wie Taschen oder Schläuche auch aus planen Geweben ausgebildet werden können.

Der Begriff "Implantat" bezeichnet ein künstliches Material, das für eine längere Verweildauer im Körper ausgelegt ist und dort Körperfunktionen messen, unterstützen oder übernehmen soll, physikalische Signale aussenden soll, Substanzen über einen längeren Zeitraum abgeben soll oder zu plastischen Zwecken dienen soll. Das Implantat im Sinne der Erfindung ist ein austauschbares Implantat. Ein austauschbares Implantat ist ein Implantat, das aufgrund seiner Funktionsweise entweder regelmäßig ersetzt oder gewartet oder aufgrund einer Fehlfunktion werden muss. Typischerweise soll dieses austauschbare Implantat anders als andere Implantate, z.B. Knochen- oder Zahnimplantate, nicht fest mit dem Körper verwachsen. Bevorzugt ist das Implantat im Sinne der Erfindung ein aktives Funktionsimplantat, bevorzugt ein Funktionsimplantat, das seine Funktion über elektrische Kontakte vermittelt. Typischerweise weist ein solches Implantat Elektroden auf, die mit Körpergeweben interagieren sollen.

Besonders bevorzugt ist das Implantat im Sinne der Erfindung ein Cochleaimplantat, ein Herzschrittmacher, eine Insulinpumpe, Arzneimitteldepot, ein Biosensor, eine Defibrillatorelektrode, ein implantierbarer Kardiodefibrillator oder eine Neurostimulationselektrode.

Abhängig von der Art des Implantats und dem dezellularisierten Gewebe kann dieses physikalisch oder chemisch am Implantat fixiert werden. Bevorzugt ist jedoch, dass das Implantat in die Gewebehülle eingebracht wird und keine zusätzliche Fixierung erfolgen muss.

Bei Cochleaimplantaten umhüllt das dezellularisierte Gewebe, insbesondere die Elektroden. Dadurch wird die Bildung von fibrotischem Gewebe in diesen Bereichen vermindert und die elektrischen Eigenschaften des Implantats verbessert. Zudem kann das dezellularisierte Gewebe als Matrix für einwachsende Neuriten dienen, insbesondere dann, wenn in diesem Bereich gemäß einer bevorzugten Ausführungsform Wachstumsfaktoren für Nervenzellen aufgebracht wurden. Die Neuriten können somit über das dezelluarisierte Gewebe den Spalt zwischen umgebenden Gewebe und Implantat an der Implantationsstelle besser überbrücken und schließlich die Elektrodenkontakten kontaktieren. Außerdem werden ungewollte Interaktionen zwischen verschiedenen Stimulationskanälen vermieden. Dies alles ist entscheidend für die Funktion des Implantats und damit für die Qualität der Wiederherstellung des Gehörs.

Ähnlich können die Hüllstrukturen aus dezellularisiertem Gewebe bei anderen Implantaten mit Elektrodenbestandteilen, z.B. Herzschrittmachern, Defibrillatorelektroden oder implantierbaren Kardiodefibrillatoren und Neurostimulationselektroden, eingesetzt werden.

Bei Arzneimitteldepots und Biosensoren könnten in das dezellularisierte Gewebe Wachstumsfaktoren für Blutgefäße mit eingebracht werden, um eine Implantat-nahe Gefäßversorgung zu erreichen.

Im Rahmen der vorliegenden Erfindung wurde vorteilhafterweise gefunden, dass dezellularisierte Gewebe als Hüllstrukturen ausgebildet werden können, die insbesondere aktive Implantate wie Cochleaimplantate oder Defibrillatorelektroden aufnehmen und umhüllen können. Werden die Implantate in diesen Hüllen aus dezellularisiertem Gewebe implantiert, kommt es aufgrund der Maskierung durch das dezellularisierte Gewebe zu verringerter Narbenbildung und weniger Abstoßungsreaktionen. Zudem können die Gewebehüllen vorteilhafterweise mit Substanzen versehen werden, die das gezielte Einwachsen von Zellen oder Zellbestandteile, z.B. Neuriten, begünstigen und somit die Funktion des Implantats zusätzlich verbessern. Durch das Fehlen von Narbengewebe und das Ausbleiben von Abstoßungsreaktionen werden zudem die negativen Einflüsse dieser Prozesse auf die Implantatfunktion, z.B. die Einflüsse auf die Elektrodenempfindlichkeit, reduziert.

Die Erfindung betrifft auch ein Implantat gemäß Anspruch 1; offenbart wird auch ein Implantat, das von einer Schicht des erfindungsgemäßen, dezellularisierten Gewebe umhüllt ist.

Die Implantate sind auch hierbei besonders bevorzugt ein Cochleaimplantat, ein Herzschrittmacher, eine Insulinpumpe, Arzneimitteldepot, ein Biosensor, eine Defibrillatorelektrode, ein implantierbarer Kardiodefibrillator oder eine Neurostimulationselektrode.

Zusätzlich betrifft die Erfindung die Verwendung eines dezellularisierten Gewebes biologischen Ursprungs gemäß Anspruch 2; offenbart wird auch eine Verwendung eines dezellularisierten Gewebes zur Umhüllung eines Implantats.

Schließlich betrifft die Erfindung ein Verfahren zur Verbesserung der Funktionalität bei gleich bleibender Biokompatibilität eines austauschbaren Implantats gemäß Anspruch 11; offenbart wird auch ein Verfahren zur Verbesserung der Funktionalität bei gleich bleibender Biokompatibilität eines austauschbaren Implantats umfassend die Schritte:
a) Bereitstellen eines dezellularisierten Gewebes biologischen Ursprungs; und
b) Umhüllen des Implantats mit diesem dezellularisierten Gewebe.

Unter "Verbesserung der Funktionalität bei gleich bleibender Biokompatibilität" ist hierbei zu verstehen, dass die Implantate mit Hülle aus dezellularisiertem Gewebe mindestens vergleichbar bioinert, biotolerant und/oder bioaktiv sind, wie das Implantat ohne Hülle. Jedoch soll sich die Funktion des Implantats verbessern. Diese Funktionsverbesserung kann in der Verbesserung der aktiven Funktion begründet sein, z.B. dadurch, dass es weniger Interferenzen durch körpereigene Reaktionen auf das Implantat gibt oder dadurch, dass die Interaktion mit Körpergeweben gezielt verbessert wird. Die Funktionsverbesserung kann aber auch schlicht in der verbesserten Austauschbarkeit des Implantats liegen.

Im Rahmen des erfindungsgemäßen Verfahrens wird ein Gewebe biologischen Ursprungs als dezellularisiertes Gewebe bereitgestellt. Diese Bereitstellung kann, muss aber nicht den Dezellularisierungsprozess unter Anwendung der zuvor beschriebenen physikalischen, chemischen und/oder enzymatischen Methoden zur Dezellularisierung umfassen. Ebenso kann das Verfahren Schritte zur Präparierung des dezellularisierten Gewebes mit biologisch aktiven Substanzen umfassen wie andernorts hierin beschrieben. Das dezellularisierte Gewebe wird anschließend in eine Hüllstruktur, z.B. eine Tasche oder einen Schlauch, gebracht. Schließlich wird ein Implantat in diese Hüllstruktur eingebracht.

Bevorzugt ist das dezellularisierte Gewebe, das im erfindungsgemäßen Verfahren eingesetzt wird, ein erfindungsgemäß dezellularisiertes Gewebe wie zuvor beschrieben.

### FIGUREN

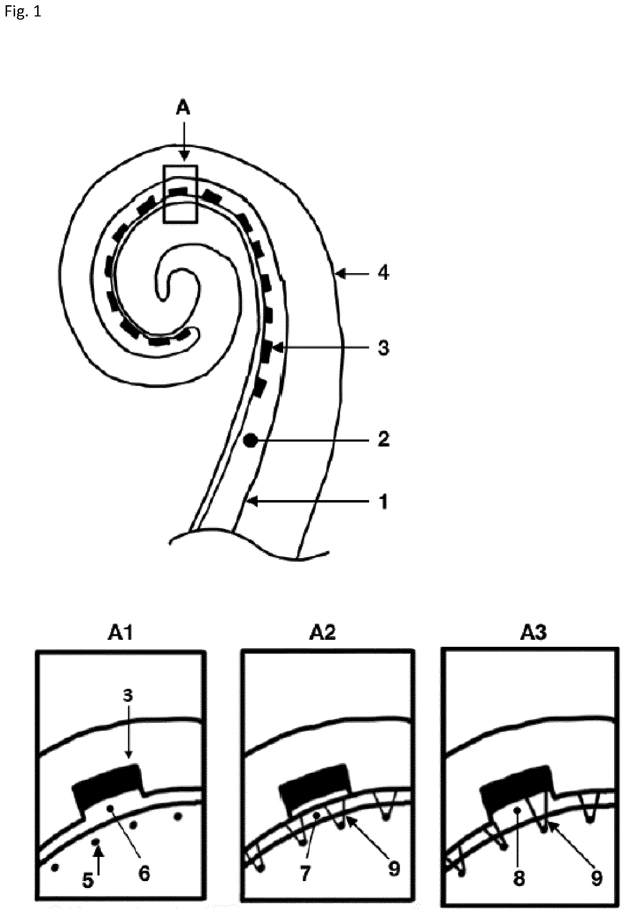

Figur 1: CI-Elektrodenträger (1) mit Silikongrundkörper (2) und Elektrodenkontakten (3) in einer Cochlea (4). Der Elektrodenkontakte sind in der Nähe der Nervenzellen (5) positioniert. Der verbleibende Spalt (6) zwischen den Elektrodenkontakten und der Nervenzellen kann durch eine Gewebehülle (7, A2) oder durch Gewebefragmente (Gewebepatches) (8, A3) geschlossen werden. Das Gewebe dient dabei als Matrix für das Wachstum von Neuriten (9).

### BEISPIELE

Die folgenden Ausführungsbeispiele illustrieren die Erfindung lediglich. Sie dürfen nicht als Einschränkungen im Hinblick auf den beabsichtigen Schutzbereiches verstanden werden.

### Beispiel 1: Dezellularisierten Geweben für Implantathüllen

Dezellularisiertes Bindegewebe wird wie in WO2006/122553 beschrieben bereitgestellt. Das Gewebe wird in Form einer Tasche für einen Herzschrittmacher oder eines Schlauches für ein Cochleaimplantat um das jeweilige Implantat gelegt. Anstelle des unmaskierten wird nun das maskierte Implantat eingesetzt. Das Körpereigene Gewebe erkennt nun das maskierte Implantat nicht mehr als Fremdkörper und es kommt z.B. nicht mehr zur Bildung einer Schicht aus Bindegewebe die das Implantat fixiert und ein explantieren erschwert. Körper-seitig verhält sich das dezellularisierte Gewebe wie bei Badylak 2011 beschrieben. Implantat-seitig kommt es zu keiner festen Verbindung.

Bei den so herzustellenden Cochleaimplantaten wird der Elektrodenträger in einen Gewebeschlauch eingehüllt. Alternativ können auch nur die Elektrodenkontakte mit Gewebepatches bedeckt. Durch diese Maskierung der Elektrodenkontakte wird die Bildung von fibrotischem Gewebe nach Implantation reduziert. Dies wird wiederum die Explantierbarkeit im Falle eines Defektes, das Restgehörvermögen (Choi 2005) und die elektrischen Eigenschaften des Implantates (Wilk 2016) verbessern.

Das Gewebe wird eine Matrix zwischen den Elektrodenkontakten und den Nervenzellen in der Achse der Cochlea bilden (Fig. 1). Durch die Überbrückung des Spalts wird ein Weg für die Neuriten der Nervenzellen geschaffen, welche so an die Elektrodenkontakte anwachsen können. Letzteres ist entscheidend für die Qualität der Gehörwiederherstellung, da große Distanzen zwischen den Elektrodenkontakten und den Nervenzellen zu ungewollten Interaktionen zwischen den Stimulationskanälen führen können.

### Zitierte Literatur

Choi 2005, Hear Res. 205(1-2):193-200.
Wilk 2016, PLoS One. 11(2):e0147552. doi: 10.1371/journal.pone.0147552. eCollection 2016.
Badylak 2011, Annu. Rev. Biomed. Eng. 2011. 13:27-53.
Tudorache 2016, European Journal of Cardio- Thoracic Surgery 50, 89-97.
Gstoettner 2001, Acta Otolaryngol 121:216-219.
Gilbert 2006, Biomaterials. 27: 3675-3683.
Crapo 2011, Biomaterials. 32: 3233-3243.
WO2006/122553.

## Patentansprüche

1. Ein Implantat, das von einer Schicht dezellularisiertem Gewebe biologischen Ursprungs umhüllt ist, wobei das Implantat ein Cochleaimplantat, ein Retinaimplantat, ein Herzschrittmacher, eine Insulinpumpe, Arzneimitteldepot, ein Biosensor, eine Defibrillatorelektrode, ein implantierbarer Kardiodefibrillator oder eine Neurostimulationselektrode ist und wobei das Implantat ein austauschbares Implantat ist.

2. Verwendung eines dezellularisierten Gewebes biologischen Ursprungs zur Umhüllung eines Implantats, wobei das Implantat ein Cochleaimplantat, ein Retinaimplantat, ein Herzschrittmacher, eine Insulinpumpe, Arzneimitteldepot, ein Biosensor, eine Defibrillatorelektrode, ein implantierbarer Kardiodefibrillator oder eine Neurostimulationselektrode ist und wobei das Implantat ein austauschbares Implantat ist.

3. Das Implantat nach Anspruch 1 oder die Verwendung nach Anspruch 2, wobei das Implantat präpariert ist, um das gezielte Einwachsen bestimmter Zellen oder Zellteile zu ermöglichen.

4. Das Implantat oder die Verwendung nach Anspruch 3, wobei die Zellen oder Zellteile Nervenzellen oder Teile davon sind.

5. Das Implantat nach Anspruch 1, 3, oder 4 oder die Verwendung nach einem der Ansprüche 2 bis 4, wobei das dezellularisierte Gewebe biologischen Ursprungs zu einer das Implantat umhüllenden Schicht ausgebildet ist und das Implantat ganz oder teilweise umhüllt.

6. Das Implantat nach einem der Ansprüche 1 und 3 bis 5 oder die Verwendung nach einem der Ansprüche 2 bis 5, wobei das dezellularisierte Gewebe biologischen Ursprungs zu einer Tasche oder einem Schlauch zur Aufnahme des Implantats ausgebildet ist.

7. Das Implantat nach einem der Ansprüche 1 und 3 bis 6 oder die Verwendung nach einem der Ansprüche 2 bis 6, wobei das dezellularisierte Gewebe biologischen Ursprungs durch Behandlung von Gewebe biologischen Ursprungs mit physikalisch, chemisch und oder enzymatischen Methoden, bevorzugt durch Behandlung mit Detergenzien und osmotisch wirksamen Lösungen, gewonnen wurde.

8. Das Implantat nach einem der Ansprüche 1 und 3 bis 7 oder die Verwendung nach einem der Ansprüche 2 bis 7, wobei das dezellularisierte Gewebe biologischen Ursprungs extrazelluläre Matrix ist oder diese umfasst.

9. Das Implantat nach einem der Ansprüche 1 und 3 bis 8 oder die Verwendung nach einem der Ansprüche 2 bis 8, wobei das dezellularisierte Gewebe biologischen Ursprungs mit anti-inflammatorisch wirksamen Substanzen versehen ist.

10. Das Implantat nach einem der Ansprüche 1 und 3 bis 9 oder die Verwendung nach einem der Ansprüche 2 bis 9, wobei das Implantat ein austauschbares Implantat ist.

11. Ein Verfahren zur Verbesserung der Funktionalität bei gleich bleibender Biokompatibilität eines austauschbaren Implantats umfassend die Schritte:
a) Bereitstellen eines dezellularisierten Gewebes biologischen Ursprungs; und
b) Umhüllen des Implantats mit diesem dezellularisierten Gewebe,
wobei das Implantat ein Cochleaimplantat, ein Retinaimplantat, ein Herzschrittmacher, eine Insulinpumpe, Arzneimitteldepot, ein Biosensor, eine Defibrillatorelektrode, ein implantierbarer Kardiodefibrillator oder eine Neurostimulationselektrode ist.

12. Verfahren nach Anspruch 11, wobei das dezellularisierte Gewebe ein Gewebe ist, wie es in einem der Ansprüche 1 bis 9 beschrieben wird.

## Claims

1. Implant which is encased by a layer of decellularized tissue of biological origin, wherein the implant is a cochlear implant, a retinal implant, a pacemaker, an insulin pump, drug depot, a biosensor, a defibrillator electrode, an implantable cardiac defibrillator or a neurostimulation electrode and wherein the implant is an exchangeable implant.

2. Use of a decellularized tissue of biological origin for encasing an implant, wherein the implant is a cochlear implant, a retinal implant, a pacemaker, an insulin pump, drug depot, a biosensor, a defibrillator electrode, an implantable cardiac defibrillator or a neurostimulation electrode and wherein the implant is an exchangeable implant.

3. Implant according to Claim 1 or use according to Claim 2, wherein the implant has been prepared to allow the specific ingrowth of specific cells or cell parts.

4. Implant or use according to Claim 3, wherein the cells or cell parts are nerve cells or parts thereof.

5. Implant according to Claim 1, 3 or 4 or use according to any of Claims 2 to 4, wherein the decellularized tissue of biological origin is designed to form a layer encasing the implant and completely or partially encases the implant.

6. Implant according to any of Claims 1 and 3 to 5 or use according to any of Claims 2 to 5, wherein the decellularized tissue of biological origin is designed to form a pocket or a tube for accommodation of the implant.

7. Implant according to any of Claims 1 and 3 to 6 or use according to any of Claims 2 to 6, wherein the decellularized tissue of biological origin was obtained by treatment of tissue of biological origin with physical, chemical and/or enzymatic methods, preferably by treatment with detergents and osmotically active solutions.

8. Implant according to any of Claims 1 and 3 to 7 or use according to any of Claims 2 to 7, wherein the decellularized tissue of biological origin is or comprises an extracellular matrix.

9. Implant according to any of Claims 1 and 3 to 8 or use according to any of Claims 2 to 8, wherein the decellularized tissue of biological origin has been provided with anti-inflammatorily active substances.

10. Implant according to any of Claims 1 and 3 to 9 or use according to any of Claims 2 to 9, wherein the implant is an exchangeable implant.

11. Method for improving the functionality of an exchangeable implant while maintaining biocompatibility, comprising the steps of:
a) providing a decellularized tissue of biological origin; and
b) encasing the implant with said decellularized tissue,
wherein the implant is a cochlear implant, a retinal implant, a pacemaker, an insulin pump, drug depot, a biosensor, a defibrillator electrode, an implantable cardiac defibrillator or a neurostimulation electrode.

12. Method according to Claim 11, wherein the decellularized tissue is a tissue as described in any of Claims 1 to 9.

## Revendications

1. Implant qui est recouvert d'une couche de tissu décellularisé d'origine biologique, l'implant étant un implant cochléaire, un implant rétinien, un stimulateur cardiaque, une pompe à insuline, un dépôt de médicament, un biocapteur, une électrode de défibrillateur, un défibrillateur cardiaque implantable ou une électrode de neurostimulation, et l'implant étant un implant remplaçable.

2. Utilisation d'un tissu décellularisé d'origine biologique pour recouvrir un implant, l'implant étant un implant cochléaire, un implant rétinien, un stimulateur cardiaque, une pompe à insuline, un dépôt de médicament, un biocapteur, une électrode de défibrillateur, un défibrillateur cardiaque implantable ou une électrode de neurostimulation, et l'implant étant un implant remplaçable.

3. Implant selon la revendication 1 ou utilisation selon la revendication 2, l'implant étant préparé pour permettre la croissance ciblée de cellules ou de parties de cellules déterminées.

4. Implant ou utilisation selon la revendication 3, les cellules ou parties de cellules étant des cellules nerveuses ou des parties de celles-ci.

5. Implant selon la revendication 1, 3 ou 4 ou utilisation selon l'une des revendications 2 à 4, le tissu décellularisé d'origine biologique étant conçu sous la forme d'une couche de recouvrement de l'implant et recouvrant complètement ou partiellement l'implant.

6. Implant selon l'une des revendications 1 et 3 à 5 ou utilisation selon l'une des revendications 2 à 5, le tissu décellularisé d'origine biologique étant conçu sous la forme d'une poche ou d'un tuyau destiné à recevoir l'implant.

7. Implant selon l'une des revendications 1 et 3 à 6 ou utilisation selon l'une des revendications 2 à 6, le tissu décellularisé d'origine biologique ayant été obtenu par traitement de tissu d'origine biologique avec des procédés physiques, chimiques et/ou enzymatiques, de préférence par traitement avec des détergents et des solutions osmotiquement actives.

8. Implant selon l'une des revendications 1 et 3 à 7 ou utilisation selon l'une des revendications 2 à 7, le tissu décellularisé d'origine biologique étant ou comprenant une matrice extracellulaire.

9. Implant selon l'une des revendications 1 et 3 à 8 ou utilisation selon l'une des revendications 2 à 8, le tissu décellularisé d'origine biologique étant pourvu de principes actifs anti-inflammatoires.

10. Implant selon l'une des revendications 1 et 3 à 9 ou utilisation selon l'une des revendications 2 à 9, l'implant étant un implant remplaçable.

11. Procédé d'amélioration de la fonctionnalité avec conservation de la biocompatibilité d'un implant remplaçable, ledit procédé comprenant les étapes suivantes :
a) fournir un tissu décellularisé d'origine biologique ; et
b) recouvrir l'implant avec ce tissu décellularisé, l'implant étant un implant cochléaire, un implant rétinien, un stimulateur cardiaque, une pompe à insuline, un dépôt de médicament, un biocapteur, une électrode de défibrillateur, un défibrillateur cardiaque implantable ou une électrode de neurostimulation.

12. Procédé selon la revendication 11, le tissu décellularisé étant un tissu tel que décrit dans l'une des revendications 1 à 9.
